(19) Europäisches Patentamt
European Patent Office
Office européen des brevets

(11) Publication number: 0 047 105 B1

# (12) EUROPEAN PATENT SPECIFICATION

(45) Date of publication of patent specification: 28.11.84

(51) Int. Cl.³: C 07 D 455/06, A 61 K 31/47

(21) Application number: 81303795.9

(22) Date of filing: 20.08.81

## (54) Benzoquinolizines, processes for their preparation and pharmaceutical compositions.

(30) Priority: 28.08.80 GB 8027889
16.04.81 GB 8112080

(43) Date of publication of application:
10.03.82 Bulletin 82/10

(45) Publication of the grant of the patent:
28.11.84 Bulletin 84/48

(84) Designated Contracting States:
AT BE CH DE FR IT LI LU NL SE

(56) References cited:
GB-A-1 513 824
GB-A-1 532 078
GB-A-2 039 276
US-A-4 183 937

(73) Proprietor: JOHN WYETH & BROTHER LIMITED
Huntercombe Lane South Taplow
Maidenhead Berkshire, SL6 0PH (GB)

(72) Inventor: Archibald, John Leheup
136, Springfield Road
Windsor Berkshire (GB)
Inventor: Ward, Terence James
11, Iona Crescent
Cippenham Slough Berkshire (GB)

(74) Representative: Brown, Keith John Symons
et al
c/o John Wyeth & Brother Limited Patent and
Trademark Department Huntercombe Lane
South
Taplow Maidenhead Berkshire SL6 0PH. (GB)

EP 0 047 105 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

This invention relates to benzoquinolizines, to processes for preparing the benzoquinolizines and to pharmaceutical preparations containing them.

GB—A—1,513,824 discloses that benqoquinolizines of the general formula (I)

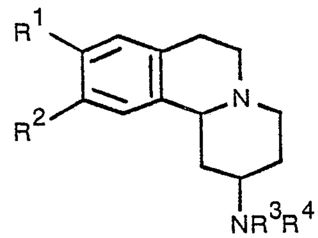

I

and the pharmaceutically acceptable acid addition salts thereof, wherein $R^1$ and $R^2$ which may be the same or different, each represent hydrogen, lower alkyl, lower alkoxy or halogen, $R^3$ represents hydrogen, lower alkyl or aryl and $R^4$ represents —$SO_2R^5$ (where $R^5$ is lower alkyl or aryl), — $CONH_2$ or —$CXNHR^6$ (where X is oxygen or sulphur and $R^6$ is aryl or aryl.CO.—), generally exhibit hypotensive activity upon administration to warm-blooded animals.

We have now found that a small group of compounds falling within general formula (I) but not specifically disclosed in the above mentioned specification, and their pharmaceutically acceptable acid addition salts possess high presynaptic $\alpha$-adrenoceptor antagonistic activity in warm blooded animals. This activity is not disclosed or foreshadowed in the above mentioned specification.

Accordingly the present invention provides benzoquinolizines of the general formula (II)

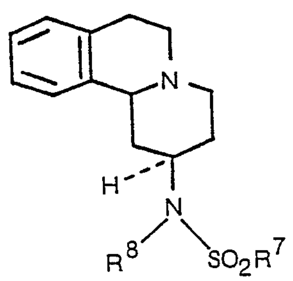

II

and the pharmaceutically acceptable acid addition salts thereof, wherein

$R^8$ is methyl or ethyl and $R^7$ is naphthyl optionally substituted by one or more $C_{1-6}$ alkyl, $C_{1-6}$ alkoxy or halogen substituents or

$R^8$ is methyl and $R^7$ is methyl, ethyl, n-propyl, n-butyl, phenyl, 4-methylphenyl, 2-methylphenyl, 4-methoxyphenyl, 4-chlorophenyl or 3,4-dichlorophenyl or

$R^8$ is ethyl and $R^7$ is methyl.

Examples of halogen substituents include fluorine, chlorine and bromine.

Preferred compounds of formula (II) are

N-methyl-N-(1,3,4,6,7,11b$\alpha$-hexahydro-2*H*-benzo[a]quinolizin-2$\beta$-yl)methanesulphonamide
N-methyl-N-(1,3,4,6,7,11b$\alpha$-hexahydro-2*H*-benzo[a]quinolizin-2$\beta$-yl)propan-1-sulphonamide
N-methyl-N-(1,3,4,6,7,11b$\alpha$-hexahydro-2*H*-benzo[a]quinolizin-2$\beta$-yl)benzenesulphonamide
and their pharmaceutically acceptable acid addition salts.

The compounds of the invention were tested for their presynaptic $\alpha$-adrenoceptor antagonistic activity on the rat field stimulated vas deferens preparation using a modification of the method of Drew, Eur. J. Pharmac., 1977, *42*, 123—130. The procedure is described below.

Desheathed vasa deferentia from sexually mature rats were suspended in a 6-ml organ bath in Krebs solution at 37° and bubbled with 5% $CO_2$ in oxygen. Platinum ring electrodes were positioned above and below the tissue for field stimulation, the stimulus parameters being 0.1 Hz 1 ms pulse width at supramaximal voltage. Twitch responses were recorded isotonically with a 0.5 g loading. Clonidine hydrochloride was used as the $\alpha$-adrenoceptor agonist and cumulative concentration-response curves were constructed for the inhibition of twitch obtained with clonidine in the range of 0.125 to 4 ng $ml^{-1}$. After washing out clonidine, the twitch response quickly recovered and an antagonist was then introduced into the Krebs reservoir. Clonidine concentration-response curves were repeated 90 min after introduction of the antagonist. The concentration of clonidine producing 50% inhibition of twitch before and after introduction of antagonist were obtained and the dose-ratio for clonidine was calculated. Various concentrations of the antagonists were used.

These results were plotted in the manner described by Arunlakshana and Schild, Br. J. Pharmac. Chemother., 1959, *14*, 48—58 and the values of $pA_2$ and slope were calculated. The compounds of the invention possess potent presynaptic $\alpha$-adrenoceptor antagonistic activity having, in general, values of $pA_2$ of 7.5 or more and substantially greater than other compounds of general formula (I), some of which are specifically exemplified in GB—A—1,513,824, as is shown in the following Table I:

TABLE I

| Compound | $pA_2$ (presynaptic) |
|---|---|
| Compounds of the invention | |
| N-methyl-N-(1,3,4,6,7,11b$\alpha$-hexahydro-2H-benzo[a]-quinolizin-2$\beta$-yl)methanesulphonamide (Example 2) | 7.7 |
| N-methyl-N-(1,3,4,6,7,11b$\alpha$-hexahydro-2H-benzo[a]-quinolizin-2$\beta$-yl)ethanesulphonamide (Example 3) | 7.7 |
| N-methyl-N-(1,3,4,6,7,11b$\alpha$-hexahydro-2H-benzo[a]-quinolizin-2$\beta$-yl)propan-1-sulphonamide (Example 4) | 8.3 |
| N-methyl-N-(1,3,4,6,7,11b$\alpha$-hexahydro-2H-benzo[a]-quinolizin-2$\beta$-yl)benzenesulphonamide (Example 5) | 8.4 |
| N-Ethyl-N-(1,3,4,6,7,11b$\alpha$-hexahydro-2H-benzo[a]-quinolizin-2$\beta$-yl)methanesulphonamide (Example 6) | 7.8 |
| N-methyl-N-(1,3,4,6,7,11b$\alpha$-hexahydro-2*H*-benzo[a] quinolizin-2$\beta$-yl)toluene-4-sulphonamide (Example 7) | 8.1 |
| N-methyl-N-(1,3,4,6,7,11b$\alpha$-hexahydro-2*H*-benzo[a]-quinolizin-2$\beta$-yl)-4-methoxybenzenesulphonamide (Example 8) | 8.1 |
| N-methyl-N-(1,3,4,6,7,11b$\alpha$-hexahydro-2*H*-benzo[a]-quinolizin-2$\beta$-yl)-4-chlorobenzenesulphonamide (Example 9) | 7.6 |
| N-methyl-N-(1,3,4,6,7,11b$\alpha$-hexahydro-2H-benzo[a]-quinolizin-2$\beta$-yl)-2-methylbenzenesulphonamide (Example 10) | 7.9 |
| N-methyl-N-(1,3,4,6,7,11b$\alpha$-hexahydro-2*H*-benzo[a]-quinolizin-2$\beta$-yl)-3,4-dichlorobenzenesulphonamide (Example 11) | 7.5 |
| N-methyl-N-(1,3,4,6,7,11b$\alpha$-hexahydro-2*H*-benzo[a]-quinolizin-2$\beta$-yl)-n-butanesulphonamide (Example 12) | 7.9 |

| Other compounds of formula I (according to GB—A—1513824) | |
|---|---|
| 2-methanesulphonamido-1,3,4,6,7,11b-hexahydro-2H-benzo[a]quinolizine | 6.9 |
| N-methyl-N-(9,10-dimethoxy-1,3,4,6,7,11b$\alpha$-hexahydro-2H-benzo-[a]quinolizin-2$\beta$-yl)-methanesulphonamide | 6.8 |
| N-(n-Propyl)-N-(1,3,4,6,7,11b$\alpha$-hexahydro-2H-benzo[a]quinolizin-2$\beta$-yl)methanesulphonamide | 7.0 |
| 2$\beta$-(n-propanesulphonamido-1,3,4,6,7,11b$\alpha$-hexahydro-2*H*-benzo[a]quinolizine | 6.5 |
| 1-(1,3,4,6,7,11b-hexahydro-2*H*-benzo[a]quinolizin-2yl)-3-phenyl urea | 5.4 |
| 1-(1,3,4,6,7,11b-hexahydro-2*H*-benzo[a]-quinolizin-2yl)-3-phenylthiourea | 5.6 |
| 2$\beta$-phenylsulphonamido-1,3,4,6,7,11b-hexahydro-2*H*-benzo[a]quinolizine | 6.4 |
| 2$\beta$-ethanesulphonamido-1,3,4,6,7,11b-hexahydro-2*H*-benzo[a]quinolizine | 5.3 |

The compounds of the invention have been found to antagonise the presynaptic $\alpha$-adrenoceptors to a much greater extent than the postsynaptic $\alpha$-adrenoceptors. The postsynaptic antagonistic activity can be evaluated by a number of different methods. One method involves assessing the activity on the isolated anococcygeus muscle of the rat. The method is based on that of Gillespie, Br. J. Pharmac., 1972, *45*, 404—416. In the procedure male rats (250—360 g) are killed by a blow on the head and bled. The two anococcygeus muscles are removed from their position in the midline of the pelvic cavity, where they arise from the upper coccygeal vertebrae. The muscles are suspended in 5 ml organ baths in Krebs solution containing $10^{-4}$M ascorbic acid, to prevent drug oxidation. The tissues are gassed with a 95% oxygen, 5% $CO_2$ mixture and maintained at 37°. Longitudinal muscle contractions are recorded using isotonic transducers. Cumulative dose response curves are then obtained to phenylephrine or in some cases methoxamine, both agents being presynaptic alpha adrenoceptor agonists. The concentration range of phenylephrine or methoxamine used is 0.02 to 0.8 $\mu$g.ml$^{-1}$. The agonist is then washed from the bath and the test drug added to the bathing medium at a concentration of $10^{-6}$M. After 30 min. equilibration with the test drug a further agonist dose response curve is obtained. The washing, equilibration and agonists dosing procedures are then repeated using $10^{-5}$M and $10^{-4}$M solutions of the test drug. Estimates of the $pA_2$ value for the test drug as an antagonist of phenylephrine or methoxamine were made from the agonist dose-ratios using the method of Arunlakshana and Schild, Br. J. Pharmac, Chemother., 1959, *14*, 48—58.

The $pA_2$ for postsynaptic antagonistic activity and the presynaptic selectivity ($pA_2$ presynaptic antagonistic activity/$pA_2$ postsynaptic antagonistic activity) for the compounds of the invention are given in Table II below.

TABLE II

| Compound of Example | $pA_2$ (postsynaptic) | Presynaptic selectivity |
|---|---|---|
| 2 | 5.95 | 56 |
| 3 | 6.17 | 34 |
| 4 | 6.14 | 144 |
| 5 | 6.34 | 102 |
| 6 | 6.25 | 35.5 |
| 7 | 6.33 | 59 |
| 8 | 6.5 | 40 |
| 9 | 6.09 | 32 |
| 10 | 6.27 | 40 |
| 11 | 5.48 | 93 |
| 12 | 6.6 | 19 |

Compounds having good presynaptic $\alpha$-adrenoceptor angatonistic activity, particularly those with a high presynaptic selectivity are potentially useful, for example, as antidepressants, in treatment of peripheral vascular disease in treatment of diabetes and in inhibiting blood platelet aggregation. In such use any substantial hypotensive activity of the compound would be an undesirable side effect. We have found unexpectantly that some of the compounds of the present invention (such as compound of Example 2 above) do not possess such acitivity when tested by a standard pharmacological procedure employing rats rendered hypertensive by implantation of desoxycorticosterone acetate.

The compounds of the present invention can be prepared by reacting a reactive derivative of a sulphonic acid of general formula (III)

$$R^7SO_2OH \qquad III$$

with a 2$\beta$-methylamino or ethylamino-1,3,4,6,7,11b-hexahydro-2*H*-benzo[a]quinolizine of general formula (IV)

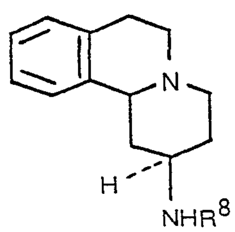

IV

when $R^7$ and $R^8$ are as defined above and, if required, converting a free base into a pharmaceutically acceptable acid addition salt. The reactive derivative of the sulphonic acid can be, for example, the acid halide or anhydride. Preferably it is the halide i.e. a compound of general formula $R^7SO_2X$ (where $R^7$ is as defined above and X is halogen, preferably chlorine). The reaction is preferably carried out under basic conditions, for example in the presence of a tertiary amine, e.g. triethylamine.

If in the process described above the compound of the invention is obtained as an acid addition salt, the free base can be obtained by basifying a solution of the acid addition salt. Conversely, if the product of the process is a free base, an acid addition salt, particularly a pharmaceutically acceptable acid addition salt, may be obtained by dissolving the free base in a suitable organic solvent and treating the solution with an acid, in accordance with conventional procedures for preparing acid addition salts from base compound.

Examples of acid addition salts are those formed from inorganic and organic salts, such as sulphuric, hydrochloric, hydrobromic, phosphoric, tartaric, fumaric, maleic, citric, acetic, formic, methanesulphonic and *p*-toluenesulphonic acids.

The 2β-ethylamino or methylamino-1,3,4,6,7,11b-hexahydro-2*H*-benzo[a]quinolizine starting materials of formula (IV) for the above process can be prepared from the corresponding 2-oxo-compound by the procedure described in GB—A—1,513,824. Alternatively the 2-methyl-amino starting material can be prepared from the corresponding 2-amino compound, e.g. by reacting the amino compound with an alkylhaloformate or with formic acid and reducing, e.g. with a hydride transfer reagent such as lithium aluminium hydride the resulting 2-$NHCO_2$Alkyl or 2-NHCHO intermediate.

The invention further provides a pharmaceutical composition comprising a compound of the invention in association with a pharmaceutically acceptable carrier. Any suitable carrier known in the art can be used to prepare the pharmaceutical composition. In such a composition, the carrier is generally a solid or liquid of a mixture of a solid and a liquid.

Solid form compositions include powders, granules, tablets, capsules (e.g. hard and soft gelatin capsules), suppositories and pessaries. A solid carrier can be, for example, one or more substances which may also act as flavouring agents, lubricants, solubilisers, suspending agents, fillers, glidants, compression aids, binders or tablet-disintegrating agents; it can also be an encapsulating material. In powders the carrier is a finely divided solid which is in admixture with the finely divided active ingredient. In tablets the active ingedient is mixed with a carrier having the necessary compression properties in suitable proportions and compacted in the shape and size desired. The powders and tablets preferably contain up to 99%, e.g. from 0.03 to 99%, preferably 1 to 80% of the active ingredient. Suitable solid carriers include, for example, calcium phosphate, starch, gelatin, cellulose, methyl cellulose, sodium carboxymethyl cellulose, polyvinylpyrrolidine, low melting waxes and ion exchange resins.

The term "composition" is intended to include the formulation of an active ingredient with encapsulating material as carrier to give a capsule in which the active ingredient (with or without other carriers) is surrounded by the carrier, which is thus in association with it. Similarly cachets are included.

Liquid form compositions include, for example, solutions, suspensions, emulsions, syrups, elixers and pressurised compositions. The active ingredient, for example, can be dissolved or suspended in a pharmaceutically acceptable liquid carrier such as water, an organic solvent, a mixture of both or pharmaceutically acceptalbe oils or fats. The liquid carrier can contain other suitable pharmaceutical additives such as solubilizers, emulsifiers, buffers, preservatives, sweeteners, flavouring agents, suspending agents, thickening agents, colours, viscosity regulators, stabilisers or osmo-regulators. Suitable examples of liquid carriers for oral and parenteral administration include water (particularly containing additives as above e.g. cellulose derivatives, preferably sodium carboxymethyl cellulose solution), alcohols (including monohydric alcohols and polyhydric alcohols e.g. glycerol and glycols) and their derivatives, and oils (e.g. fractionated coconut oil and arachis oil). For parenteral administration the carrier can also be an oily ester such as ethyl oleate and isopropyl myristate. Sterile liquid carriers are used in sterile liquid form compositions for parenteral administration.

Liquid pharmaceutical compositions which are sterile solutions or suspensions can be utilised by, for example, intramuscular, intraperitoneal or subcutaneous injection. Sterile solutions can also be administered intravenously. When the compound is orally active it can be administered orally either in liquid or solid composition form.

Preferably the pharmaceutical composition is in unit dosage form, e.g. as tablets or capsules. In such form, the composition is sub-divided in unit dose containing appropriate quantities of the active ingredient; the unit dosage forms can be packaged compositions, for example packeted powders, vials, ampoules, prefilled syringes or sachets containing liquids. The unit dosage form can be, for example, a capsule or tablet itself, or it can be the appropriate number of any such compositions in package form. The quantity of the active ingredient in a unit dose of composition may be varied or adjusted from 0.5 mg or less to 750 mg or more, according to the particular need and the activity of the active ingredient. The invention also includes the compounds in the absence of the carrier where the compounds are in unit dosage form.

The following Example 1 illustrates the preparation of a starting material for preparing the compounds of the invention. Examples 2 to 12 illustrate the compounds of the invention and their preparation.

Example 1 (starting material)

2β-Methylamino-1,3,4,6,7,11bα-hexahydro-2*H*-benzo[a]quinoline

(a) 2β-Amino-1,3,4,6,7,11bα-hexahydro-2*H*-benzo[a]quinolizine (17.7 g) was cooled to 0° (ice) and cautiously treated with 100% formic acid (100 $cm^3$). The mixture was stirred until homogenous, then slowly treated with acetic anhydride (10.0 g) over about 1 hour at 0°. After stirring for a further 1 hour, the mixture was heated to reflux for 2 hours, cooled and excess solvent was evaporated in vacuo. The residual oil was basified with 10% aq.$Na_2CO_3$ (ca.500 $cm^3$) and extracted with dichloromethane (3 × 100 $cm^3$). The combined extracts were washed with brine (100 $cm^3$) and dried ($MgSO_4$). Glc at this stage revealed incomplete reaction (ca. 50%), and the entire cycle was repeated with fresh formic

acid and acetic anhydride. Evaporation of the organic extract gave a mixture of solid and oil (16.4 g). Crystallisation from ethanol-60/80 petrol afforded pure N-[1,3,4,6,7,11b$\alpha$-hexahydro-2*H*-benzo-(a)-quinolizin-2$\beta$-yl]formamide (7.12 g) as off-white microneedles, mp 162—4° (partial decomposition occurs above 147°).

(b) A suspension of the product of part (a) (7.3 g) in dry THF (80 $cm^3$) was added slowly to a solution of lithium aluminium hydride (4 g) in dry THF (120 $cm^3$). The mixture was stirred and heated to reflux for 24 hours under a blanket of dry nitrogen, then decomposed by the dropwise addition of water (4 $cm^3$), 15% aq. NaOH (4 $cm^3$) and water (12 $cm^3$). After filtration, the solution was evaporated, the residue taken up in dichloromethane, dried ($MgSO_4$), filtered and evaporated to give a red-brown oil (6.19 g) virtually pure by tlc, which set solid on standing.

A sample (0.57 g) was converted to the hydrochloride in ethanol (7 $cm^3$) and ethanolic hydrogen chloride. On cooling, crystals were obtained which were filtered off, washed with 1:2 ethanol/ethyl acetate and dried in vacuo to give pure 2$\beta$-methylamino-1,3,4,6,7-11b$\alpha$-hexahydro-2*H*-benzo[a]-quinolizine hydrochloride (0.67 g; 88.2%) as colourless micro-needles, mp 248—251°(dec.) (fast rate of heating; decomposition begins at temperatures above 200°).

## Example 2

N-Methyl-N-(1,3,4,6,7,11b$\alpha$-hexahydro-2*H*-benzo[a]quinolizin-2$\beta$-yl)methanesulphonamide

2$\beta$-Methylamino-1,3,4,6,7,11b$\alpha$-hexahydro-2*H*-benzo[a]quinolizine (1.88 g) and triethylamine (0.90 g) were dissolved in ice-cold dichloromethane (10 $cm^3$) and treated with an ice-cold solution of methane sulphonyl chloride (1.00 g) in dichloromethane (5 $cm^3$). The clear solution was allowed to stand overnight. The resulting mixture of crystals and solution was diluted with dichloromethane (10 $cm^3$) and water (5 $cm^3$), and extracted with water (2 × 20 $cm^3$). The organic layer was separated and dried ($MgSO_4$). Filtration and evaporation gave a pink gum (2.37 g). The hydrochloride was prepared from ethanolic hydrogen chloride an recrystallized from ethanol-ethyl acetate, to yield the title compound hydrochloride as off-white micro-needles (1.45 g), mp 198—200° (dec).

## Example 3

N-Methyl-N-(1,3,4,6,7,11b$\alpha$-hexahydro-2*H*-benzo[a]quinolizin-2$\beta$-yl)ethanesulphonamide

A solution of ethanesulphonyl chloride (1.45 g) in dichloromethane (20 $cm^3$) was added slowly to an ice-cold solution of 2$\beta$-methylamino-1,3,4,6,7,11b$\alpha$-hexahydro-2*H*-benzo[a]quinolizine and triethylamine (1.2 g; 0.0119 M) in dichloromethane (30 $cm^3$). The clear mixture was stirred briefly, then kept at room temperature for 7 days, when tlc showed that the reaction was complete. It was washed with water (2 × 25 $cm^3$) and dried ($MgSO_4$). Filtration and evaporation afforded a yellow-orange syrup (3.13 g) which was dissolved in hot ethanol (5 $cm^3$), acidified with ethanolic hydrogen chloride, diluted with ethyl acetate (15 $cm^3$) and cooled. The precipitated, sticky crystals were filtered off and washed with 1:3 ethanol/ethyl acetate. Two recrystallisations from 1:1 ethanol/ethyl acetate afforded the title compound as the hydrochloride (0.97 g), colourless crystals, mp 207—212° (dec) (decomposition causes local melting above 180°).

## Example 4

N-Methyl-N-(1,3,4,6,7,11b$\alpha$-hexahydro-2H-benzo[a]quinolizin-2$\beta$-yl)propan-1-sulphonamide

An ice-cold, stirred solution of 2$\beta$-methylamino-1,3,4,6,7,11b$\alpha$-hexahydro-2*H*-benzo[a]-quinolizine (2.16 g) and triethylamine (1.1 g) in dichloromethane (25 $cm^3$) was slowly treated with a solution of n-propanesulphonyl chloride (1.43 g) in dichloromethane (25 $cm^3$). The clear solution was then kept at room temperature for 72 h, when tlc showed complete reaction had occurred. The mixture was washed with water (2 × 25 $cm^3$), dried ($MgSO_4$), filtered and evaporated to give a viscous, pale-yellow oil (3.09 g). This was dissolved in hot ethanol (7 $cm^3$), acidified with ethanolic hydrogen chloride, diluted with ethyl acetate (25 $cm^3$) and cooled, with scratching. The crystals which slowly separated were collected by filtration, washed with 20% ethanol-ethyl acetate and recrystallized from methanol to give the title compound as the hydrochloride (1.98 g), colourless plates, m.p. approx. 222—240°C (dec. violent decomposition occurring above 200° without melting).

## Example 5

N-Methyl-N-(1,3,4,6,7,11b$\alpha$-hexahydro-2H-benzo[a]quinolizin-2$\beta$-yl)benzenesulphonamide

An ice-cold, stirred solution of 2$\beta$-methylamino-1,3,4,6,7,11b$\alpha$-hexahydro-2*H*-benzo[a]quinolizine and triethylamine (0.7 g) in dichloromethane (25 $cm^3$) was slowly treated with a solution of benzenesulphonyl chloride (1.2 g) in dichloromethane (25 $cm^3$). The clear solution was kept at room temperature for 72 h when tlc showed complete reaction had occurred. The mixture was washed with water (2 × 25 $cm^3$), dried ($MgSO_4$), filtered and evaporated to give an off-white glass (2.44 g). This was dissolved in ethanol (7 $cm^3$), acidified with ethanolic HCl, diluted with ethyl acetate (10 $cm^3$) and cooled. The crystals were filtered off and washed with 1:1 ethanol/ethyl acetate to give the title compound as the hydrochloride (2.32 g), colourless needles, m.p. 225—228° (dec, partial decomposition occurring above 193°).

## Example 6

N-Ethyl-N-(1,2,3,4,6,7,11bα-hexahydro-2H-benzo[a]quinolizin-2β-yl)methanesulphonamide

An ice-cold solution of 2-ethylamino-1,3,4,6,7,11b-hexahydro-2H-benzo[a]quinolizine (1.35 g) and triethylamine (0.60 g) in dichloromethane (50 $cm^3$) was treated, with stirring, with a solution of dimesyl anhydride (1.02 g) in dichloromethane (25 $cm^3$). The mixture was stirred for a further 2 h, then allowed to stand at room temperature until tlc indicated that reaction was virtually complete (11 days). The mixture was then washed with water (2 x 25 $cm^3$), dried ($MgSO_4$), filtered and evaporated to give an oil (1.59 g). Chromatography on silica eluted with 5—10% ethanol/ethyl acetate afforded a purified product (0.88 g) which was acidified with ethanolic hydrogen chloride and, after evaporation of the solvent, crystallized from iso-propanol. The crystals were recrystallised from boiling ethanol (3 $cm^3$) to which ethyl acetate (5 $cm^3$) was added, to give pure title compound as the hydrochloride quaterhydrate (0.55 g), colourless crystals, m.p. 217—223° (dec) (with sublimation and partial decomposition above 200°).

## Example 7

N-Methyl-N-(1,3,4,6,7,11bα-hexahydro-2*H*-benzo[a]quinolizin-2β-yl)toluene-4-sulphonamide

A stirred solution of 2β-methylamino-1,3,4,6,7,11bα-hexahydro-2*H*-benzo[a]quinolizine (2.16 g and triethylamine (1.1 g) in dichloromethane (25 $cm^3$) was cooled in ice and treated with a solution of tosyl chloride (1.91 g) in dichloromethane (25 $cm^3$). The clear mixture was kept at room temperature for 6 days, then washed with water (2 x 25 $cm^3$) and dried ($MgSO_4$). Filtration and evaporation afforded a red-orange glass (3.69 g) which crystallised from hot ethanol (10 $cm^3$). After cooling, the crystals were filtered off and washed with ice-cold ethanol to give the title compound (4.43 g) as cream crystals, m.p. 161—164°.

Trituration with boiling ethanol and acidification with ethanolic HCl (which caused dissolution followed by re-precipitation), followed by stirring to break up lumps and filtration afforded the pure *hydrochloride* (3.39 g) as light buff rods, m.p. 258—261° (dec; decomposition occurred at all temperature above 220°).

## Example 8

N-Methyl-N-(1,3,4,6,7,11bα-hexahydro-2*H*-benzo[a]quinolizin-2β-yl)-4-methoxybenzenesulphonamide

An ice-cold, stirred solution of 2β-methylamino-1,3,4,6,7,11bα-hexahydro-2*H*-benzo[a]quinolizine (2.16 g) and triethylamine (1.1 g) in dichloromethane (25 $cm^3$) was treated with a solution of p-methoxybenzenesulphonyl chloride (2.07 g) in dichloromethane (25 $cm^3$). After standing for 7 days, the solution was washed with water (2 x 25 $cm^3$), dried ($MgSO_4$) and evaporated to give a residue (3.61 g) which was crystallised from ethanol (15 $cm^3$) to give the title compound as cream crystals, m.p. 154—6°.

The base was triturated with boiling ethanol (10 $cm^3$), acidified with ethanolic HCl (which caused partial dissolution followed by reprecipitation), cooled and stirred well to break up lumps. Filtration gave off-white crystals which were triturated well with a boiling mixture of ethanol/methanol/water. After cooling, the crystals were filtered off and washed well with ethanol, to give the title compound as the hydrochloride (2.62 g) as pale buff crystals with no clear m.p. (decomp. occurs above 220°).

## Example 9

N-Methyl-N-(1,3,4,6,7,11bα-hexahydro-2*H*-benzo[a]quinolizin-2β-yl)-4-chlorobenzenesulphonamide

An ice-cold, stirred solution of 2β-methylamino-1,3,4,6,7,11bα-hexahydro-2*H*-benzo[a]quinolizine (1.08 g) and triethylamine (0.55 g) in dichloromethane (25 $cm^3$) was treated with a solution of 4-chlorobenzenesulphonyl chloride (1.06 g) in dichloromethane. The mixture was kept at room temperature for 7 days, then washed with water (2 x 25 $cm^3$), dried ($MgSO_4$), filtered and evaporated to give the title compound as a brown semi-crystalline gum (1.64 g) which crystallised from hot ethanol (5 $cm^3$) to give pale-buff crystals, m.p. 50—3° (dec).

The crystals were taken up in boiling ethanol (10 $cm^3$), acidfied with ethanolic HCl (causes partial dissolution followed by re-precipitation), cooled and stirred well. Filtration gave very pale pink crystals which were triturated with a hot ethanol/methanol/water mixture, cooled, filtered and washed with ethanol to give the title compound hydrochloride (1.10 g), colourless, short plates, with no clear m.p. (decomp. occurs above 230°).

## Example 10

N-Methyl-N-(1,3,4,6,7,11bα-hexahydro-2H-benzo[a]quinolizin-2β-yl)-2-methylbenzenesulphonamide

An ice-cold, stirred solution of 2β-methylamino-1,3,4,6,7,11bα-hexahydro-2H-benzo[a]quinolizine (1.84 g) and triethylamine (0.9 g) in dichloromethane (25 $cm^3$) was slowly treated with a solution of *o*-toluenesulphonyl chloride (1.62 g) in dichloromethane (25 $cm^3$). The solution was kept at room temperature for 2 days, then washed with water (2 x 50 $cm^3$) and dried ($MgSO_4$). Filtration and

evaporation afforded an orange syrup (3.37 g) which was dissolved in hot ethanol (10 $cm^3$), acidified with ethanolic HCl and cooled. The crystals were filtered off after 1 hour, and washed with ethanol to give the title compound as the hydrochloride (2.88 g) as pale cream crystals. Recrystallisation from ethanol/water gave the pure hydrochloride, quarterhydrate (2.03 g) as colourless needles, m.p. 197—203° (dec; decomp. occurred above 175°).

Example 11

N-Methyl-N-(1,3,4,6,7,11b$\alpha$-hexahydro-2*H*-benzo[a]quinolizin-2$\beta$-yl)-3,4-dichlorobenzene-sulphonamide

An ice-cold, stirred solution of 2$\beta$-methylamino-1,3,4,6,7,11b$\alpha$-hexahydro-2*H*-benzo[a]quinolizine (1.84 g) and triethylamide (0.9 g) in dichloromethane (25 $cm^3$) was slowly treated with a solution of 3,4-dichlorobenzenesulphonyl chloride (2.09 g) in dichloromethane (25 $cm^3$). The mixture was kept at room temperature for 2 days, then washed with water (2 x 50 $cm^3$) and dried ($MgSO_4$). Filtration and evaporation afforded an off-white solid (3.30 g) which was purified by trituration with hot ethanol, to give pure title compound, m.p. 191—3°.

A suspension of the sulphonamide in boiling ethanol (10 $cm^3$) was acidified with ethanolic HCl and the clear solution cooled. Filtration and washing with ethanol gave the hydrochloride (3.03 g) as pale cream crystals. Recrystallisation from ethanol/water afforded the title compound as the pure hydrochloride, quarterhydrate (2.71 g), as colourless crystals, m.p. 195—197° (dec).

Example 12

N-Methyl-N-(1,3,4,6,7,11b$\alpha$-hexahydro-2*H*-benzo[a]quinolizin-2$\beta$-yl)-n-butanesulphonamide

An ice-cold stirred solution of 2$\beta$-methylamino-1,3,4,6,7,11b$\alpha$-hexahydro-2*H*-benzo[a]-quinolizine (2.16 g) and triethylamine (1.2 g) in dichloromethane (25 $cm^3$) was treated with a solution of n-butanesulphonyl chloride (1.57 g) in dichloromethane (25 $cm^3$). The clear solution was kept at room temperature for 4 days, washed with water (2 x 50 $cm^3$) and dried ($MgSO_4$). Filtration and evaporation afforded a dark gum (2.96 g) which was chromatographed on silica eluted with 10% ethanol-ethyl acetate to give a pale yellow syrup (2.04 g). This was dissolved in hot ethanol (5 $cm^3$), acidified with ethanolic HCl, diluted with ethyl acetate (20 $cm^3$) and cooled. After several hours, the precipitated crystals were filtered, washed with ethyl acetate and dried at 60°/100 mm to give the title compound as the hydrochloride (2.25 g), colourless microplates, m.p. 224—226° (dec).

**Claims for the Contracting States: BE CH DE FR IT LI LU NL SE**

1. A benzoquinolizine of the general formula (II)

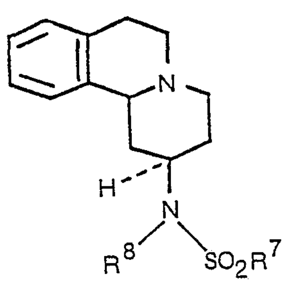

II

or a pharmaceutically acceptable acid addition salt thereof, wherein

$R^8$ is methyl or ethyl and $R^7$ is naphthyl optionally substituted by one or more $C_{1-6}$ alkyl, $C_{1-6}$ alkoxy or halogen substituents or

$R^8$ is methyl and $R^7$ is methyl, ethyl, n-propyl, n-butyl, phenyl, 4-methylphenyl, 2-methylphenyl, 4-methoxyphenyl, 4-chlorophenyl or 3,4-dichlorophenyl or

$R^8$ is ethyl and $R^7$ is methyl.

2. N-Ethyl-N-(1,3,4,6,7,11b$\alpha$-hexahydro-2*H*-benzo[a]quinolizin-2$\beta$-yl)methanesulphonamide or a pharmaceutically acceptable acid addition salt thereof.

3. N-Methyl-N-(1,3,4,6,7,11b$\alpha$-hexahydro-2*H*-benzo[a]quinolizin-2$\beta$-yl)methanesulphonamide or a pharmaceutically acceptable acid addition salt thereof.

4. N-Methyl-N-(1,3,4,6,7,11b$\alpha$-hexahydro-2*H*-benzo[a]quinolizin-2$\beta$-yl)propan-1-sulphonamide or a pharmaceutically acceptable acid addition salt thereof.

5. N-Methyl-N-(1,3,4,6,7,11b$\alpha$-hexahydro-2*H*-benzo[a]quinolizin-2$\beta$-yl)benzenesulphonamide or a pharmaceutically acceptable acid addition salt thereof.

6. N-Methyl-N-(1,3,4,6,7,11b$\alpha$-hexahydro-2*H*-benzo[a]quinolizin-2$\beta$-yl)ethanesulphonamide or a pharmaceutically acceptable acid addition salt thereof.

7. A compound according to Claim 1 which is N-methyl-N-(1,3,4,6,7,11bα-hexahydro-2*H*-benzo[a]quinolizin-2β-yl)toluene-4-sulphonamide, N-methyl-N-(1,3,4,6,7,11bα-hexahydro-2*H*-benzo[a]quinolizin-2β-yl)-4-methoxybenzenesulphonamide, N-methyl-N-(1,3,4,6,7,11bα-hexahydro-2*H*-benzo[a]quinolizin-2β-yl)-4-chlorobenzenesulphonamide, N-methyl-N-(1,3,4,6,7,11bα-hexahydro-2*H*-benzo[a]quinolizin-2β-yl)-2-methylbenzenesulphonamide, N-methyl-N-(1,3,4,6,7,11bα-hexahydro-2*H*-benzo[a]quinolizin-2β-yl)-3,4-dichlorobenzenesulphonamide or N-methyl-N-(1,3,4,6,7,11bα-hexahydro-2*H*-benzo[a]quinolizin-2β-yl-n-butanesulphonamide or a pharmaceutically acceptable acid addition salt thereof.

8. A process for preparing a compound claimed in any one of Claims 1 to 7 which comprises reacting a reactive derivative of a sulphonic acid of general formula (III)

$$R^7SO_2OH \qquad III$$

with 2β-methylamino- or ethylamino-1,3,4,6,7,11bα-hexahydro-2*H*-benzo[a]quinolizine of general formula (IV)

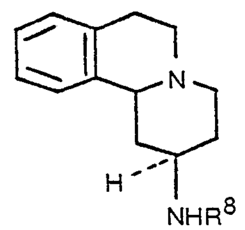

IV

where $R^7$ and $R^8$ are as defined in Claim 1 and, if required converting a free base into a pharmaceutically acceptable acid addition salt.

9. A pharmaceutical composition having presynaptic α-adrenoceptor antagonistic activity comprising a benzoquinolizine as claimed in any one of Claims 1 to 7, in association with a pharmaceutically acceptable carrier.

10. A compound as claimed in any one of Claims 1 to 7 for use in in antagonising presynaptic α-adrenoceptors in warm blooded animals.

**Claims for the Contracting State: AT**

1. A process for preparing a benzoquinolizine of the general formula (II)

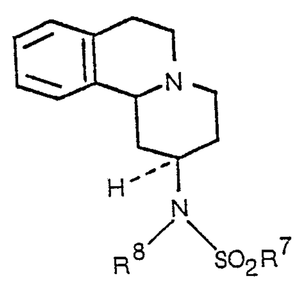

II

or a pharmaceutically acceptable acid addition salt thereof, wherein

$R^8$ is methyl or ethyl and $R^7$ is naphthyl optionally substituted by one or more $C_{1-6}$ alkyl, $C_{1-6}$ alkoxy or halogen substituents or

$R^8$ is methyl and $R^7$ is methyl, ethyl, n-propyl, n-butyl, phenyl, 4-methylphenyl, 2-methylphenyl, 4-methoxyphenyl, 4-chlorophenyl or 3,4-dichlorophenyl or

$R^8$ is ethyl and $R^7$ is methyl,

which comprises reacting a reactive derivative of a sulphonic acid of general formula (III)

$$R^7SO_2OH \qquad III$$

with 2β-methylamino- or ethylamino-1,3,4,6,7,11bα-hexahydro-2*H*-benzoquinolizine or general formula (IV)

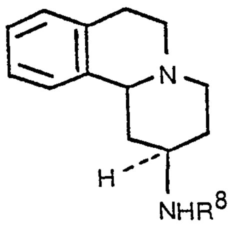

IV

where $R^7$ and $R^8$ are as defined above, and if required, converting a free base into a pharmaceutically acceptable acid addition salt.

2. A process as claimed in Claim 1 wherein the reactive derivative of the sulphonic acid is an acid halide or anhydride.

3. A process for preparing a pharmaceutically acceptable acid addition or a benzoquinolizine of general formula (II) as defined in Claim 1 which comprises reacting the base of general formula (II) with an inorganic or organic acid.

4. A process according to any one of Claims 1 to 3 wherein the product is N-methyl-N-(1,3,4,6,7,11bα-hexahydro-2*H*-benzo[a]quinolizin-2β-yl)methanesulphonamide or a pharmaceutically acceptable acid addition salt thereof.

5. A process according to any one of Claims 1 to 3 wherein the product is N-methyl-N-(1,3,4,6,7,11bα-hexahydro-2*H*-benzo[a]quinolizin-2β-yl)propan-1-sulphonamide or a pharmaceutically acceptable acid addition salt thereof.

6. A process according to any one of Claims 1 to 3 wherein the product is N-methyl-N-(1,3,4,6,7,11bα-hexahydro-2*H*-benzo[a]quinolizin-2β-yl)benzenesulphonamide or a pharmaceutically acceptable acid addition salt thereof.

7. A process according to any one of Claims 1 to 3 wherein the product is N-methyl-N-(1,3,4,6,7,11bα-hexahydro-2*H*-benzo[a]quinolizin-2β-yl)ethanesulphonamide or a pharmaceutically acceptable acid addition salt thereof.

8. A process according to any one of Claims 1 to 3 wherein the product is N-ethyl-N-(1,3,4,6,7,11bα-hexahydro-2*H*-benzo[a]quinolizin-2β-yl)methanesulphonamide, N-methyl-N-(1,3,4,6,7,11bα-hexahydro-2*H*-benzo[a]quinolizin-2β-yl)toluene-4-sulphonamide, N-methyl-N-(1,3,4,6,7,11bα-hexahydro-2*H*-benzo[a]quinolizin-2β-yl)-4-methoxybenzenesulphonamide, N-methyl-N-(1,3,4,6,7,11bα-hexahydro-2*H*-benzo[a]quinolizin-2β-yl)-4-chlorobenzenesulphonamide, N-methyl-N-(1,3,4,6,7,11bα-hexahydro-2*H*-benzo[a]quinolizin-2β-yl)-2-methylbenzenesulphonamide, N-methyl-N-(1,3,4,6,7,11bα-hexahydro-2*H*-benzo[a]quinolizin-2β-yl)-3,4-dichlorobenzenesulphonamide or N-methyl-N-(1,3,4,6,7,11bα-hexahydro-2*H*-benzo[a]quinolizin-2β-yl-n-butanesulphonamide or a pharmaceutically acceptable acid addition salt thereof.

9. A process for preparing a pharmaceutical composition having presynaptic α-adrenoceptor antagonistic activity which comprises bringing into association with a pharmaceutically acceptable carrier a compound of the formula (II)

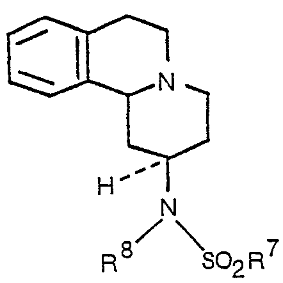

II

(wherein $R^7$ and $R^8$ are as defined in Claim 1) or a pharmaceutically acceptable acid addition salt thereof.

10. A process as claimed in Claim 9 wherein the benzoquinolizine is prepared by a process claimed in any one of Claims 1 to 3.

**Patentansprüche für die Vertragsstaaten: BE CH DE FR IT LI LU NL SE**

1. Ein Benzochinolizin der allgemeinen Formel (II)

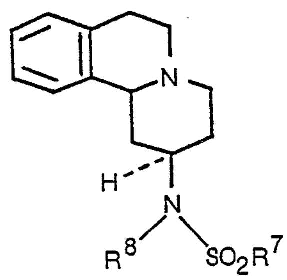

II

oder ein pharmazeutisch annehmbares Säureadditionssalz hievon, worin $R^8$ Methyl oder Äthyl und $R^7$ Naphthyl gegebenenfalls substituiert durch einen oder mehrere $C_{1-6}$-Alkyl-, $C_{1-6}$-Alkoxy- oder Halogensubstituenten bedeuten oder $R^8$ Methyl und $R^7$ Methyl, Äthyl, n-Propyl, n-Butyl, Phenyl, 4-Methylphenyl, 2-Methylphenyl, 4-Methoxyphenyl, 4-Chlorphenyl oder 3,4-Dichlorphenyl darstellen oder $R^8$ Äthyl und $R^7$ Methyl sind.

2. N-Äthyl-N-(1,3,4,6,7,11bα-hexahydro-2H-benzo[a]chinolizin-2β-yl)-methansulfonamid oder ein pharmazeutisch annehmbares Säureadditionssalz hievon.

3. N-Methyl-N-(1,3,4,6,7,11bα-hexahydro-2H-benzo[a]chinolizin-2β-yl)-methansulfonamid oder ein pharmazeutisch annehmbares Säureadditionssalz hievon.

4. N-Methyl-N-(1,3,4,6,7,11bα-hexahydro-2H-benzo[a]chinolizin-2β-yl)-propan-1-sulfonamid oder ein pharmazeutisch annehmbares Säureadditionssalz hievon.

5. N-Methyl-N-(1,3,4,6,7,11bα-hexahydro-2H-benzo[a]chinolizin-2β-yl)-benzolsulfonamid oder ein pharmazeutisch annehmbares Säureadditionssalz hievon.

6. N-Methyl-N-(1,3,4,6,7,11bα-hexahydro-2H-benzo[a]chinolizin-2β-yl)-äthansulfonamid oder ein pharmazeutisch annehmbares Säureadditionssalz hievon.

7. Eine Verbindung gemäß Anspruch 1, die N-Methyl-N-(1,3,4,6,7,11bα-hexahydro-2H-benzo[a]-chinolizin-2β-yl)-toluol-4-sulfonamid, N-Methyl-N-(1,3,4,6,7,11bα-hexahydro-2H-benzo[a]chinolizin-2β-yl)-4-methoxybenzolsulfonamid, N-Methyl-N-(1,3,4,6,7,11bα-hexahydro-2H-benzo[a]chinolizin-2β-yl)-4-chlorbenzolsulfonamid, N-Methyl-N-(1,3,4,6,7,11bα-hexahydro-2H-benzo[a]chinolizin-2β-yl)-2-methylbenzolsulfonamid, N-Methyl-N-(1,3,4,6,7,11bα-hexahydro-2H-benzo[a]chinolizin-2β-yl)-3,4-dichlobenzolsulfonamid oder N-Methyl-N-(1,3,4,6,7,11bα-hexahydro-2H-benzo[a]chinolizin-2β-yl)-n-butansulfonamid oder ein pharmazeutisch annehmbares Säureadditionssalz hievon ist.

8. Verfahren zum Herstellen einer Verbindung, wie in einem der Ansprüche 1 bis 7 beansprucht, welches das Umsetzen eines reaktionsfähigen Derivats einer Sulfonsäure der allgemeinen Formel (III)

$$R^7SO_2OH \qquad (III)$$

mit 2β-Methylamino- oder -Äthylamino-1,3,4,6,7,11bα-hexahydro-2H-benzo[a]chinolizin der allgemeinen Formel (IV)

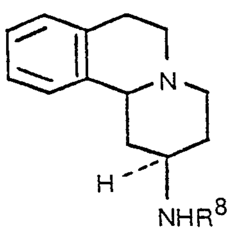

IV

worin $R^7$ und $R^8$ wie in Anspruch 1 definert sind, und, wenn erforderlich, Überführen einer freien Base in ein pharmazeutisch annehmbares Säureadditionssalz umfaßt.

9. Pharmazeutische Zusammensetzung mit präsynaptischer α-adrenozeptor-antagonistischer Wirksamkeit, die ein Benzochinolizin, wie in einem der Ansprüche 1 bis 7 beansprucht, Vereinigung mit einem pharmazeutisch annehmbaren Träger aufweist.

10. Eine Verbindung, wie in einem der Ansprüche 1 bis 7 beansprucht, zur Verwendung in antagonisierenden präsynaptischen α-Adrenozeptoren in Warmblütern.

**Patentansprüche für den Vertragsstaat: AT**

1. Verfahren zum Herstellen eines Benzochinolizins der allgemeinen Formel

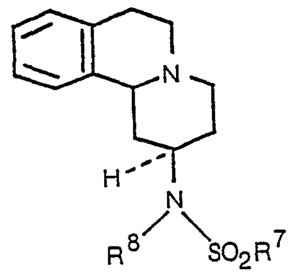

II

oder eines pharmazeutisch annehmbaren Säureadditionssalzes hievon, worin $R^8$ Methyl oder Äthyl und $R^7$ Naphthyl gegebenenfalls substituiert durch einen oder mehrere $C_{1-6}$-Alkyl-, $C_{1-6}$-Alkoxy- oder Halogensubstituenten bedeuten oder $R^8$ Methyl und $R^7$ Methyl, Äthyl, n-Propyl, n-Butyl, Phenyl, 4-Methylphenyl, 2-Methylphenyl, 4-Methoxyphenyl, 4-Chlorphenyl oder 3,4-Dichlorphenyl darstellen oder $R^8$ Äthyl und $R^7$ Methyl sind, welches das Umsetzen eines reaktionsfähigen Derivates einer Sulfonsäure der allgemeinen Forml (III)

$$R^7SO_2OH \qquad (III)$$

mit 2$\beta$-Methylamino- oder -Äthylamino-1,3,4,6,7,11b$\alpha$-hexahydro-2H-benzochinolizin der allgemeinen Formel (IV)

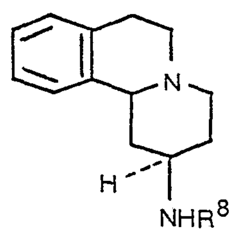

IV

worin $R^7$ und $R^8$ wie oben definiert sind, und, wenn erforderlich, Überführen einer freien Base in ein pharmazeutisch annehmbares Säureadditionssalz umfaßt.

2. Verfahren, wie in Anspruch 1 beansprucht, worin das reaktions-fähige Derivat der Sulfonsäure ein Säurehalogenid oder -anhydrid ist.

3. Verfahren zum Herstellen eines pharmazeutisch annehmbaren Säureadditionssalzes eines Benzochinolizins der allgemeinen Formel (II), wie in Anspruch 1 definiert, welches das Umsetzen der Base der allgemeinen Formel (II) mit einer anorganischen oder organischen Säure umfaßt.

4. Verfahren gemäß einem der Ansprüche 1 bis 3, worin das Produkt N-Methyl-N-(1,3,4,6,7,11b$\alpha$-hexahydro-2H-benzo[a]chinolizin-2$\beta$-yl)-methansulfonamid oder ein pharmazeutisch annehmbares Säureadditionssalz hievon ist.

5. Verfahren gemäß einem der Ansprüche 1 bis 3, worin das Produkt N-Methyl-N-(1,3,4,6,7,11b$\alpha$-hexahydro-2H-benzo[a]chinolizin-2$\beta$-yl)-propan-1-sulfonamid oder ein pharmazeutisch annehmbares Säureadditionssalz hievon ist.

6. Verfahren gemäß einem der Ansprüche 1 bis 3, worin das Produkt N-Methyl-N-(1,3,4,6,7,11b$\alpha$-hexahydro-2H-benzo[a]chinolizin-2$\beta$-yl)-benzolsulfonamid oder ein pharmazeutisch annehmbares Säureadditionssalz hievon ist.

7. Verfahren gemäß enem der Ansprüche 1 bis 3, worin das Produkt N-Methyl-N-(1,3,4,6,7,11b$\alpha$-hexahydro-2H-benzo[a]chinolizin-2$\beta$-yl)-äthansulfonamid oder ein pharmazeutisch annehmbares Säureadditionssalz hievon ist.

8. Verfahren gemäß einem der Ansprüch 1 bis 3, worin das Produkt N-Äthyl-N-(1,3,4,6,7,11b$\alpha$-hexahydro-2H-benzo[a]chinolizin-2$\beta$-yl)-methansulfonamid, N-Methyl-N-(1,3,4,6,7,11b$\alpha$-hexahydro-2H-benzo[a]chinolizin-2$\beta$-yl)-toluol-4-sulfonamid, N-Methyl-N-(1,3,4,6,7,11b$\alpha$-hexahydro-2H-benzo[a]chinolizin-2$\beta$-yl)-4-methoxybenzolsulfonamid, N-Methyl-N-(1,3,4,6,7,11b$\alpha$-hexahydro-2H-benzo[a]chinolizin-2$\beta$-yl)-4-chlorobenzolsulfonamid, N-Methyl-N-(1,3,4,6,7,11b$\alpha$-hexahydro-2H-benzo[a]chinolizin-2$\beta$-yl)-2-methylbenzolsulfonamid, N-Methyl-N-(1,3,4,6,7,11b$\alpha$-hexahydro-2H-benzo[a]chinolizin-2$\beta$-yl)-3,4-dichlorobenzolsulfonamid oder N-Methyl-N-(1,3,4,6,7,11b$\alpha$-hexahydro-2H-benzo[a]chinolizin-2$\beta$-yl)-n-butansulfonamid oder ein pharmazeutisch annehmbares Säureadditionssalz hievon ist.

9. Verfahren zum Herstellen einer pharmazeutischen Zusammensetzung mit präsynaptischer $\alpha$-

adrenozeptor-antagonistischer Wirksamkeit, welches das Vereinigen einer Verbindung der Formel (II)

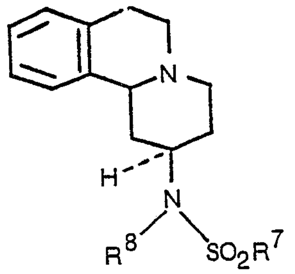

II

(worin $R^7$ und $R^8$ wie in Anspruch 1 definiert sind) oder eines pharmazeutisch annehmbaren Säureadditionssalzes hievon mit einem pharmazeutisch annehmbaren Träger umfaßt.

10. Verfahren, wie in Anspruch 9 beansprucht, worin das Benzochinolizin nach einem Verfahren, wie in einem der Ansprüche 1 bis 3 beansprucht, hergestellt ist.

**Revendications pour les Etats contractants: BE CH DE FR IT LI LU NL SE**

1. Benzoquinolizine de formule générale (II)

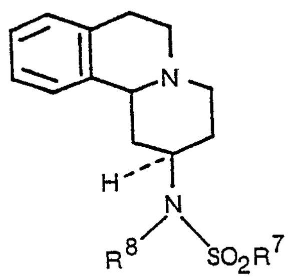

II

ou sel d'addition d'acide pharmaceutiquement acceptable de ce composé, formule dans laquelle

$R^8$ est un groupe méthyle ou éthyle et $R^7$ est un groupe napthyle éventuellement substitué par un ou plusieurs substituants alkyle en $C_1$ à $C_6$, alkoxy en $C_1$ à $C_6$ ou halogéno, ou bien

$R^8$ est un groupe méthyle et $R^7$ est un groupe méthyle, éthyle, n-propyle, n-butyle, phényle, 4-méthylephényle, 2-méthylphényle, 4-méthoxyphényle, 4-chlorophényle ou 3,4-dichlorophényle, ou bien

$R^8$ est un groupe éthyle et $R^7$ est un groupe méthyle.

2. Le N-éthyl-N-(1,3,4,6,7,11bα-hexahydro-2*H*-benzo[a]quinolizine-2β-yl)méthanesulfamide ou un sel d'addition d'acide pharmaceutiquement acceptable de ce composé.

3. Le N-méthyl-N-(1,3,4,6,7,11bα-hexahydro-2*H*-benzo[a]quinolizine-2β-yl)méthanesulfamide ou un sel d'addition d'acide pharmaceutiquement acceptable de ce composé.

4. Le N-méthyl-N-(1,3,4,6,7,11bα-hexahydro-2*H*-benzo[a]quinolizine-2β-yl)propane-1-sulfamide ou un sel d'addition d'acide pharmaceutiquement acceptable de ce composé.

5. Le N-méthyl-N-(1,3,4,6,7,11bα-hexahydro-2*H*-benzo[a]quinolizine-2β-yl)benzènesulfamide ou un sel d'addition d'acide pharmaceutiquement acceptable de ce composé.

6. Le N-méthyl-N-(1,3,4,6,7,11bα-hexahydro-2*H*-benzo[a]quinolizine-2β-yl)éthanesulfamide ou un sel d'addition d'acide pharmaceutiquement acceptable de ce composé.

7. Composé suivant la revendication 1, qui est le N-méthyl-N-(1,3,4,6,7,11bα-hexahydro-2*H*-benzo[a]quinolizine-2β-yl)toluène-4-sulfamide, le N-méthyl-N-(1,3,4,6,7,11bα-hexahydro-2*H*-benzo[a]quinolizine-2β-yl)-4-méthoxybenzènesulfamide, le N-méthyl-N-(1,3,4,6,7,11bα-hexahydro-2*H*-benzo[a]quinolizine-2β-yl)-4-chlorobenzènesulfamide, le N-méthyl-N-(1,3,4,6,7,11bα-hexahydro-2*H*-benzo[a]quinolizine-2β-yl)-2-méthylbenzènesulfonamide, le N-méthyl-N-(1,3,4,6,7,11bα-hexahydro-2*H*-benzo[a]quinolizine-2β-yl)-3,4-dichlorobenzènesulfamide ou le N-méthyl-N-(1,3,4,6,7,11bα-hexahydro-2*H*-benzo[a]quinolizine-2β-yl)-n-butanesulfamide ou un sel d'addition d'acide pharmaceutiquement acceptable de ce composé.

8. Procédé de préparation d'un composé suivant l'une quelconque des revendications 1 à 7, qui consiste à faire réagir un dérivé réactif d'un acide sulfonique de formule générale (III)

$$R^7SO_2OH$$

avec une 2$\beta$-méthylamino- ou éthylamino-1,3,4,6,7,11b$\alpha$-hexahydro-2*H*-benzo[a]quinolizine de formule générale (IV)

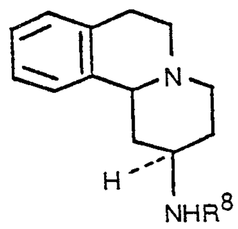

IV

dans laquelle $R^7$ et $R^8$ ont les définitions donnés dans la revendication 1 et, le cas échéant, à convertir une base libre en un sel d'addition d'acide pharmaceutiquement acceptable.

9. Composé pharmaceutique douée d'acitivité d'antagonisation des $\alpha$-adrénocepteurs présynaptiques, comprenant une benzoquinolizine suivant l'une quelconque des revendications 1 à 7 en association avec un excipient pharmaceutiquement acceptable.

10. Composé suivant l'une quelconque des revendications 1 à 7, destiné à être utilisé pour antagoniser les $\alpha$-adrénocepteurs présynaptiques chez des animaux à sang chaud.

**Revendications pour l'Etat contractant: AT**

1. Procédé de préparation d'une benzoquinolizine de formule générale (II)

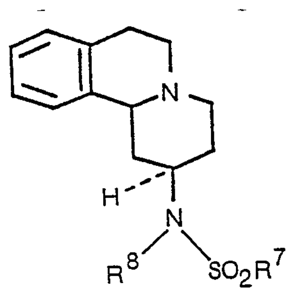

II

ou d'un sel d'addition d'acide pharmaceutiquement acceptable de ce composé, formule dans laquelle

$R^8$ est un groupe méthyle ou éthyle et $R^7$ est un groupe naptyle éventuellement substitué par un ou plusieurs substituants alkyle en $C_1$ à $C_6$, alkoxy en $C_1$ à $C_6$ ou halogéno, ou bien

$R^8$ est un groupe méthyle et $R^7$ est un groupe méthyle, éthyle, n-propyle, n-butyle, phényle, 4-méthylephényle, 2-méthylphényle, 4-méthoxyphényle, 4-chlorophényle ou 3,4-dichlorophényle, ou bien

$R^8$ est un groupe éthyle et $R^7$ est un groupe méthyle, qui consiste à faire réagir un dérivé réactif d'un acide sulfonique de formule générale (III)

$$R^7SO_2OH \qquad III$$

avec la 2$\beta$-méthylamino- ou éthylamino-1,3,4,6,7,11b$\alpha$-hexahydro-2*H*-benzoquinolizine de formule générale (IV)

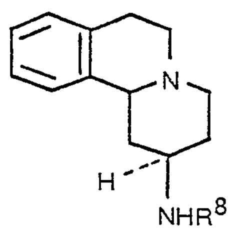

IV

dans laquelle $R^7$ et $R^8$ ont les définitions données ci-dessus et, le cas échéant, à convertir une base libre en un sel d'addition d'acide pharmaceutiquement acceptable.

2. Procédé suivant la revendication 1, dans lequel le dérivé réactif de l'acide sulfonique est un halogénure ou un anhydride d'acide.

3. Procédé de préparation d'un sel d'addition d'acide pharmaceutiquement acceptable d'une benzoquinolizine de formule générale (II) telle que définie dans la revendication 1, qui consiste à faire réagir la base de formule générale (II) avec un acide inorganique ou organique.

4. Procédé suivant l'une quelconque des renvendications 1 à 3, dans lequel le produit est le N-méthyl-N-(1,3,4,6,7,11b$\alpha$-hexahydro-2*H*-benzo[a]quinolizine-2$\beta$-yl)méthanesulfamide ou un sel d'addition d'acide pharmaceutiquement acceptable de ce composé.

5. Procédé suivant l'une quelconque des revendications 1 à 3, dans lequel le produit est le N-méthyl-N-(1,3,4,6,7,11b$\alpha$-hexahydro-2*H*-benzo[a]quinolizine-2$\beta$-yl)propane-1-sulfamide ou un sel d'addition d'acide pharmaceutiquement acceptable de ce composé.

6. Procédé suivant l'une quelconque des revendications 1 à 3, dans lequel le produit est un N-méthyl-N-(1,3,4,6,7,11b$\alpha$-hexahydro-2*H*-benzo[a]quinolizine-2$\beta$-yl)benzènesulfamide ou un sel d'addition d'acide pharmaceutiquement acceptable de ce composé.

7. Procédé suivant l'une quelconque des revendications 1 à 3, dans lequel le produit est le N-méthyl-N-(1,3,4,6,7,11b$\alpha$-hexahydro-2*H*-benzo[a]quinolizine-2$\beta$-yl)éthanesulfamide ou un sel d'addition d'acide pharmaceutiquement acceptable de ce composé.

8. Procédé suivant l'une quelconque des revendications 1 à 3, dans lequel le produit est le N-éthyl-N-(1,3,4,6,7,11b$\alpha$-hexahydro-2*H*-benzo[a]quinolizine-2$\beta$-yl)méthanesulfamide, le N-méthyl-N-(1,3,4,6,7,11b$\alpha$-hexahydro-2*H*-benzo[a]quinolizine-2$\beta$-yl)toluène-4-sulfamide, le N-méthyl-N-(1,3,4,6,7,11b$\alpha$-hexahydro-2*H*-benzo[a]quinolizine-2$\beta$-yl)-4-méthoxybenzènesulfamide, le N-méthyl-N-(1,3,4,6,7,11b$\alpha$-hexahydro-2*H*-benzo[a]quinolizine-2$\beta$-yl)-4-chlorobenzènesulfamide, le N-méthyl-N-(1,3,4,6,7,11b$\alpha$-hexahydro-2*H*-benzo[a]quinolizine-2$\beta$-yl)-2-méthylbenzènesulfamide, le N-méthyl-N-(1,3,4,6,7,11b$\alpha$-hexahydro-2*H*-benzo[a]quinolizine-2$\beta$-yl)-3,4-dichlorobenzènesulfamide ou le N-méthyl-N-(1,3,4,6,7,11b$\alpha$-hexahydro-2*H*-benzo[a]quinolizine-2$\beta$-yl)-n-butanesulfamide ou un sel d'addition d'acide pharmaceutiquement acceptable de ce composé.

9. Procédé de préparation d'une composition pharmaceutique douée d'activé antagonisante des $\alpha$-adrénocepteurs présynaptiques, qui consiste à associer avec un excipient pharmaceutiquement acceptable un composé de formule (II)

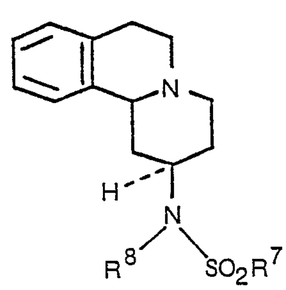

II

(dans laquelle $R^7$ et $R^8$ ont les définitions données dans la revendication 1) ou un sel d'addition d'acide pharmaceutiquement acceptable de ce composé.

10. Procédé suivant la revendication 9, dans lequel la benzoquinolizine est préparée par un procédé suivant l'une quelconque des revendications 1 à 3.